# EUROPEAN PATENT SPECIFICATION

(11) **EP 1 578 267 B1**
(45) Date of publication and mention of the grant of the patent: **26.03.2008**
(21) Application number: 03772510.8
(22) Date of filing: 21.11.2003
(51) Int. Cl.: A61B 5/103, A61B 5/0408, A61B 5/04, A61B 5/0265, A61B 5/113

(54) **FABRIC-INTEGRATED CONDUCTIVITY SENSOR**
IM GEWEBE INTEGRIERTER SENSOR ZUM MESSEN DER LEITFÄHIGKEIT
CAPTEUR DE CONDUCTIVITE INTEGRE DANS UN TISSU

(30) Priority: 19.12.2002 EP 02080429
(43) Date of publication of application: 28.09.2005
(73) Proprietor: Koninklijke Philips Electronics N.V., 5621 BA Eindhoven (NL); Philips Intellectual Property & Standards GmbH, 20099 Hamburg (DE)
(72) Inventor: PERKUHN, Michael, NL-5656 AA Eindhoven (NL); SCHMIDT, Ralf, NL-5656 AA Eindhoven (NL); REITER, Harald, NL-5656 AA Eindhoven (NL); LAUTER, Josef, NL-5656 AA Eindhoven (NL); SUCH, Olaf, NL-5656 AA Eindhoven (NL); REICHINGER, Christian, NL-5656 AA Eindhoven (NL); MONTVAY, Andras, NL-5656 AA Eindhoven (NL); MUEHLSTEFF, Jens, NL-5656 AA Eindhoven (NL)
(74) Representative: Schouten, Marcus Maria
(86) International application number: PCT/IB2003/005338
(87) International publication number: WO 2004/056268

(56) References cited:
- WO-A-01/87143
- WO-A-99/07281
- GB-A- 2 116 725
- US-A- 5 313 942
- US-A- 6 047 203
- US-A1- 2002 032 386

## Description

The invention relates to a system for monitoring a condition of a user, said system comprising:
- sensing means arranged to be located in a vicinity of a target volume of the user for detecting information representative to the user's condition, said sensing means comprising magnetic means arranged as a resonant circuit, said magnetic means being arranged to induce an oscillating magnetic field in said target volume,
- power supply means connectable to said magnetic means, said power supply means being arranged to provide a signal characteristic to a power loss of said resonant circuit upon an application of said oscillating magnetic field to said target volume,
- detection means actuatable by said sensing means, said detection means being arranged to process said signal in order to derive said information.

The invention still further relates to sensing means.

A system as set forth in the opening paragraph is known from WO 99/07281. The known system is arranged to measure an electrical conductivity in a volume under investigation by means of an application of an oscillating magnetic field to said volume. In case the volume under investigation comprises a conductor, eddy currents are generated in said volume according to principles of electromagnetic induction. A power loss due to the eddy currents induced in the conductor is a measure of the conductivity in the volume. An embodiment of the known system is arranged to measure the conductivity in a volume of a living organism. For this purpose the system is provided with at least one conductive coil sized to fit about a body of a person, an oscillator connected to the conductive coil arranged as a resonant circuit, an impedance measuring circuit arranged to provide a signal related to a power loss of said resonant circuit when applied to said living organism. In order to arrange the conductive coil around the body of the person, the known system is provided with a rigid sleeve carrier. The conductors of the known system are attached to said sleeve carrier by means of mechanical connectors. In another embodiment of the known system the conductive coil is sandwiched between an inner sleeve and an outer sleeve in order to protect the person against rigid edges of the outer sleeve.

Other measuring or monitoring systems are known from the following patent documents: WO 99/07281A, WO 01/87143A, US 5313942, US 6047203, GB 2116725A. A disadvantage of the known systems is that it accommodates the person in a not very comfortable way. The system known from WO 99/07281 A for example is arranged to fit tightly to the body of the person. The known sleeve carrier is arranged as a rigid body requiring an extra inner sleeve carrier to cushion against various rigid portions of the sleeve. It is therefore uncomfortable for the person to wear such system. In other systems, such as described in WO 01/87143 and US 5313942, the person to be examined has to be sit on a chair. Furtheron, the known systems are less suitable for purposes of durable application.

It is an object of the invention to provide a system where the usage of the system is improved. In particular, it is a further object of the invention to provide a system for durable patient monitoring.

This object is achieved according to the invention by a system for monitoring a condition of a user, said system comprising:
- sensing means arranged to be located in a vicinity of a target volume of the user for detecting information representative to the user's condition, said sensing means comprising magnetic means arranged as a resonant circuit, said magnetic means being arranged to induce an oscillating magnetic field in said target volume, the magnetic means being integrated into an insulating fabric carrier, which is a part of a bed sheet,
- power supply means connectable to said magnetic means, said power supply means being arranged to provide a signal characteristic to a power loss of said resonant circuit upon an application of said oscillating magnetic field to said target volume,
- detection means actuatable by said sensing means, said detection means being arranged to process said signal in order to derive said information

The system for monitoring a condition of a user according to the invention is particularly suited to enable durable monitoring. Due to the fact that the sensing means of the system according to the invention is integrated into a fabric carrier, the person can be permanently monitored without inducing any discomfort to that person. Next to this, due to the underlying physical principle, the monitoring can be contactlessly arranged resulting in the absence of skin irritation or other physiological discomfort of the user. The principle of the contactless measurement of conductivity in a living body by means of magnetic induction is known per se from'Contactless measurement of thoracic conductivity changes by magnetic induction', R. Guardo et al Proceedings 19th International Conference, IEEE/EMBS Oct. 30,1997.

The system according to the invention is characterized in that the magnetic means are integrated into an insulating fabric carrier, which is a part of a bed sheet, enabling a comfortable durable monitoring during user's rest. This is particularly suited for monitoring of immobilized patients.

The technical measure of the invention is based on the insight that when the magnetic means comprising resonant circuit are integrated into a fabric carrier a more mechanically flexible system for measuring conductivity in a volume can be produced.

An embodiment of the system according to the invention is characterized in that the system comprises further magnetic means arranged as a further resonant circuit, said further magnetic means being arranged in a vicinity of a further volume in order to provide a reference signal. In applications where a reference signal is required the system according to the invention is adapted with further magnetic means to induce eddy currents in a further volume yielding a reference signal. For example, the further magnetic means can be located next to the volume under investigation. Alternatively, said further magnetic means can be remotely located, for example to enable a suitable comparison between like structures.

A still further embodiment of the system for monitoring according to the invention is characterized in that the further sensing means comprise further magnetic means arranged as a resonant circuit, said further magnetic means being arranged to induce an oscillating magnetic field in said further volume. Frequently, for purposes of medical monitoring a reference signal from a healthy tissue is desirable. For example, the system according to the invention is suited to monitor a development in the cure of abnormalities, like swollen parts, oedema, injuries or the like. In cases, when such abnormality is present in an extremity, a lateral healthy extremity can provide a reference signal for purposes of comparison. In this case the system for monitoring according to the invention can be integrated into bandages to be put around the volume comprising the abnormality and a corresponding lateral volume. According to this technical measure an individualized information about a progress in the cure can be obtained.

For purposes of human monitoring is it preferable to provide an additional sensor arranged to interact with the system for monitoring, as for some medical circumstances more than one signal from the body of the user is required. For example, the further sensing means can comprise a temperature meter, a blood pressure meter or any other suitable sensor.

According to still another embodiment of the invention, the system further comprises alarm means actuatable by the detection means, said alarm means being arranged to trigger an alarm signal upon detection of said information by the detection means. Preferably, such a system comprises transmission means arranged to transmit the alarm signal to a remote station responsive to said alarm signal. In case a life threatening abnormality occurs the alarm means actuate an alarm to warn the bystanders upon an occurrence of said abnormality.

Due to the fact that the sensing means of the system according to the invention are integrated into an insulating fabric, said insulating fabric can easily be integrated not only into bed sheets, but also into cloths (T-shirt, a brassiere, or any other suitable body-wear), a furniture piece, an invalid chair, a safety belt of a vehicle or the like, etc.

When the alarm system according to the invention is integrated into a safety belt, the engine of the vehicle cam be switched off automatically upon an occurrence of a disorder in the condition of the user. The functioning of alarm systems arranged to monitor living organism are known per se and will not be explained in detail.

For individuals suffering from a life threatening condition it is preferable that the monitoring system targeting said condition is durably worn causing minimum user discomfort.

For example, for industrial applications a versatile conductivity analyzer can be produced fitting to a plurality of spatial geometries. Preferably, for industrial applications the system according to the invention is integrated into pipe coatings or the like to enable an easy monitoring or control of fluids running in such pipes. Preferably, the fabric is stretchable so that the system according to the invention is adaptable to a variety of pipe diameters.

These and other aspects of the invention will be described with reference to figures.
Fig. 1 presents schematically an embodiment of sensing means comprising a resonant circuit according to the invention.
Fig. 2 schematically illustrates an interaction between the sensing means of the system according to the invention and a conducting volume being investigated.
Fig. 3 presents schematically an embodiment of a system for monitoring according to the invention where the magnetic means are integrated into clothing.
Fig. 4 presents schematically an embodiment of a system for monitoring according to the invention, said system comprising further sensing means.
Fig. 5 presents schematically an embodiment of the alarm system according to the invention.

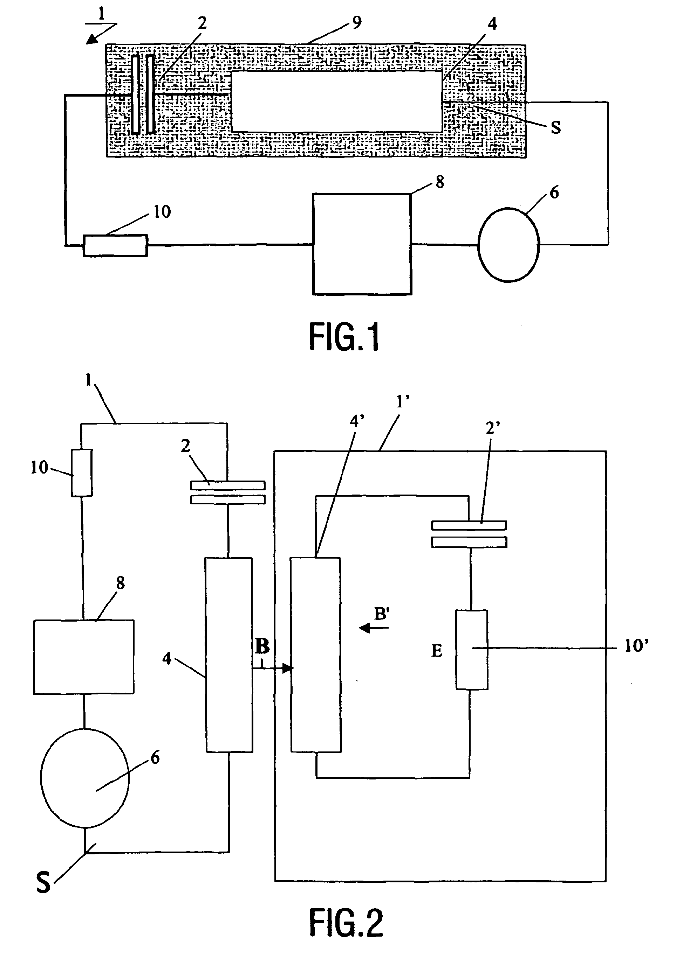

Fig. 1 presents schematically an embodiment of sensing means (1) comprising a resonant circuit 2,4,10 according to the invention. The resonant circuit comprises a resistor 10 consecutively connected to a capacitance 2 and a coil 4. Power supply means 8 energise the resonant circuit 10, 2, 4 so that an oscillating magnetic field (not shown) is produced. The signal S from the resonant circuit 10,2,4 is detected by an amperemeter 6. The power loss experienced by the resonant circuit due to an electromagnetic interaction with a conductive body (not shown) is reflected in a change in the magnitude of the signal S. By detecting the signal S the power loss by the resonant circuit is determined. In case the relation between the absolute value of the power loss and the signal S is known, the conductive characteristics of the volume being investigated can be determined. In order to ensure a constant power load, the resonant circuit preferably is enabled with a feedback loop (not shown). The feedback loop is preferably arranged so that the voltage controlling the amplitude of the resonant circuit is proportional to the RF power delivered by the resonant circuit. The resonant circuit is integrated into an insulating fabric carrier 9. Preferably, the conductors forming the coil 4 are interwoven with threads of fabric 9. In the simplest embodiment the resonant circuit comprises a single coil 4 with a single loop. In more sophisticated embodiments it is possible to design the resonator circuit with a plurality of coils comprising a plurality of loops.

Fig. 2 schematically illustrates an interaction between the sensing means of the system according to the invention and a conducting volume being investigated. When the resonant circuit 1 is activated and is brought in contact with a volume comprising a conductive body 1' processes described by Faraday's law occur. The RF current circulating in the coil 4 produces a time-varying magnetic field B, the primary field, which induces an electric field E in the volume. Due to the fact that the coil 4 is near the conducting body 1', the electric field E generates eddy currents (not shown) in that conducting body. The density of these eddy currents is proportional to the conductivity of the conducting body 1'. The induced eddy currents generate a secondary magnetic field B', pointing in the opposite direction with respect to the primary magnetic field B. Due to Faraday's law the secondary magnetic field B' induces an electromotive force in the coil 4, whose phase is 180 degrees relative to the driving current. The conductive body can thus be represented as a resistive load with an equivalent circuit comprising a capacitor 2', a coil 4' and a resistor 10'. Because the internal impedance of the conductive body is finite, any change in load resistance due to a change in conductivity will cause the amplitude of the measured signal S to vary. When the characteristics of the primary resonator circuit are known, the conductivity of the volume under investigation can thus be determined. In a human body the conductive medium is blood. Therefore, monitoring of a blood flow in a volume located in a vicinity of a resonator circuit is feasible. This is particularly suitable for cardiac applications. Alternatively, it is possible to monitor respiration rate, as during inhalation the conductivity of thorax decreases due to air inflow. The monitoring of oedema or injury healing is based on the insight that the conductivity of the volume comprising said abnormalities changes with respect to a healthy tissue due to an increase of fluid around the abnormality.

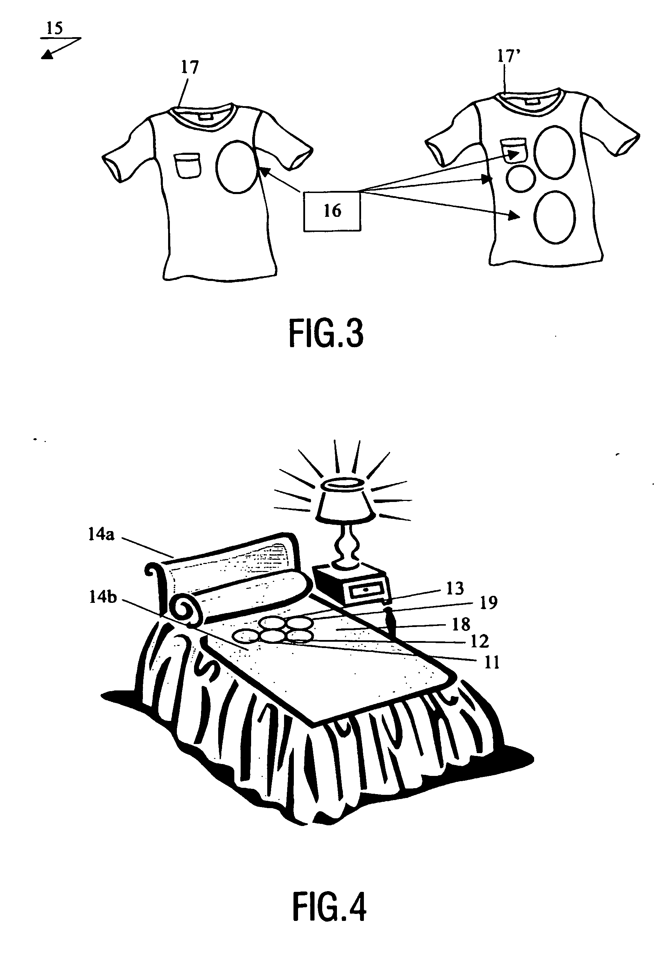

Fig. 3 presents schematically an embodiment of a system for monitoring 15according to the invention where the magnetic means are integrated into clothing. In a simplest embodiment a T-shirt 17, 17' is used as an insulating fabric carrier to be integrated with the resonator circuit 16. Here the resonator circuit 16 comprises all units discussed with reference to Fig. 1. In an alternative embodiment 17', the T-shirt is provided with a plurality of resonating coils for reference purposes.

Fig. 4 presents schematically an embodiment of a system for monitoring according to the invention, said system comprising further sensing means. In this embodiment a bed 14a is used to accommodate a person (not shown). The bed sheet 14b is provided with a plurality of sensing means 11, 12, 13, 18, 19. Preferably, two sensing means comprise a resonant circuit, as discussed with reference to figure 1. It is also possible that other sensing means are also included into the bed sheet, like a temperature meter, environment meter, like oxygen meter, respiration meter. It must be realised that the embodiment shown in Figure 4 serves only for purposes of illustration and is not intended to limit the scope of protection to any particular sensor in any way.

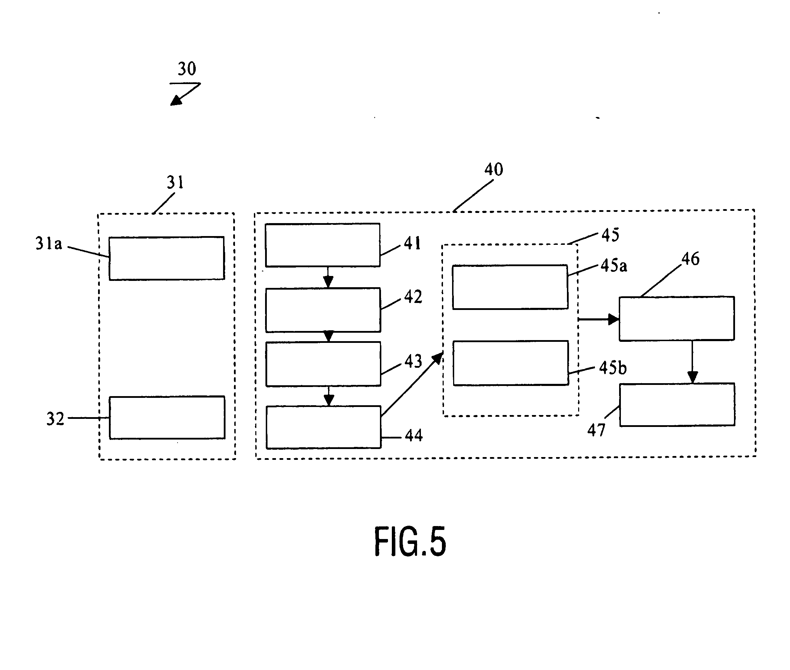

Fig. 5 presents schematically an embodiment of the alarm system according to the invention. The alarm system 30 comprises magnetic means 31 arranged to monitor a physiological condition of the user. The magnetic means 31 comprise a resonant circuit 31a to be arranged on the body of the user to pick-up a signal characteristic of the targeted physiological condition, for example a signal related to a blood flow. Additionally, the magnetic means 31 can comprise a further magnetic means 32 arranged to monitor a reference signal. The magnetic means 31 are arranged to perform a continuous monitoring of the physiological condition of the user and are further arranged to provide a corresponding signal to the front-end electronics 40 of the system 30. The magnetic means 31 and the front-end electronics 40 are worn on the body of the user, preferably at the thorax area. Alternatively, the magnetic means 31 can be integrated into a furniture peace, a bed sheet, a safety belt, etc. Examples of suitable fabric carriers for the wearable device are known per se in the art. The front-end electronics 40 is arranged to analyze the signal from the resonant circuit 31a. For that purpose the front-end electronics 40 comprises a preamplifier 41 and analogue processing circuit 42, an ADC unit 43, detection means 45 and a µ-processor 44. The front-end electronics 40 further comprises alarm means 46 and transmission means 47. The detection means 45 comprises a sensor signal interpretation unit 45a and feature extraction means 45b. The system 30 operates as follows: the magnetic means 31 acquires the raw data, which are delivered to the front-end electronics 40. The front-end electronics 40 provides means for receiving the signals from the magnetic means, performs suited analogue processing by means of the analogue processing circuit 42. The processed raw data is converted into a digital format by means of the ADC 43 and is forwarded by a µ-processor 44 to the detection means 45, where the condition of the user is being analyzed. The detection means 45 comprise a sensor signal interpretation unit 45a arranged to derive a feature in the signal characteristic of an abnormal physiological condition of the user. For cardiac applications, for example said feature can be an amplitude of the signal. In case the detection means 45 detects the abnormal condition, a signal is sent to the alarm means 46 to generate an alarm, which is transmitted by the transmitting means 47, for example by means of a RF-link to warn a bystander or specialized medical personnel.

## Claims

1. A system for monitoring a condition of a user, said system comprising:
- sensing means arranged to be located in a vicinity of a target volume of the user for detecting information representative to the user's condition, said sensing means comprising magnetic means arranged as a resonant circuit, said magnetic means being arranged to induce an oscillating magnetic field in said target volume,
- power supply means connectable to said magnetic means, said power supply means being arranged to provide a signal characteristic to a power loss of said resonant circuit upon an application of said oscillating magnetic field to said target volume,
- detection means actuatable by said sensing means, said detection means being arranged to process said signal in order to derive said information.
**characterized in that** the magnetic means are integrated into an insulating fabric carrier, which is part of a bed sheet.

2. A system according to claim 1, **characterized in that** the system comprises further sensing means arranged to be located in a vicinity of a further volume in order to provide a reference signal.

3. A system according to claim 2, **characterized in that** the further sensing means comprise further magnetic means arranged as a resonant circuit, said further magnetic means being arranged to induce an oscillating magnetic field in said further volume.

4. A system according to any one of the preceding claims 1 to 3, **characterised in that** the target volume comprises a heart of the user.

5. A system according to any one of the preceding claims 1 to 4, **characterized in that** the system further comprises alarm means actuatable by the detection means, said alarm means being arranged to trigger an alarm signal upon detection of said information by the detection means.

6. A system according to claim 5, said system comprising transmission means arranged to transmit the alarm signal to a remote station responsive to said alarm signal.

7. Sensing means for use in a system according to any one of the preceding claims, said sensing means comprise magnetic means arranged as a resonant circuit, said magnetic means being conceived to induce an oscillating magnetic field in a volume under investigation, said sensing means being further conceived to be connectable to a power supply means, **characterized in that** said sensing means are integrated into an insulating fabric carrier, which is a part of a bed sheet.

8. Sensing means according to claim 7, wherein said fabric carrier comprises threads of fabric, **characterized in that** the magnetic means comprise a loop of a conductive material, said conductive material being interwoven with said threads of fabric.

## Patentansprüche

1. System zum Überwachen eines Zustands eines Benutzers, wobei das System Folgendes umfasst:
- Sensormittel, die ausgebildet sind, in einer Umgebung eines Zielvolumens des Benutzers platziert zu werden, um Informationen zu detektieren, die für den Zustand des Benutzers repräsentativ sind, wobei die Sensormittel als Resonanzschaltung ausgebildete magnetische Mittel umfassen, wobei die magnetischen Mittel ausgebildet sind, in dem genannten Zielvolumen ein oszillierendes Magnetfeld zu induzieren,
- Leistungszufuhrmittel, die mit den genannten magnetischen Mitteln verbunden werden können, wobei die Leistungszufuhrmittel ausgebildet sind, ein Signal bereitzustellen, das für einen Leistungsverlust der genannten Resonanzschaltung beim Anlegen des oszillierenden Magnetfeldes an das Zielvolumen charakteristisch ist,
- Detektionsmittel, die von den genannten Sensormitteln betätigt werden können, wobei die Detektionsmittel ausgebildet sind, das genannte Signal zu verarbeiten, um die genannten Informationen abzuleiten,
**dadurch gekennzeichnet, dass** die magnetischen Mittel in einen isolierenden Gewebeträger integriert sind, der Teil eines Bettlakens ist.

2. System nach Anspruch 1, **dadurch gekennzeichnet, dass** das System weitere Sensormittel umfasst, die ausgebildet sind, in einer Umgebung eines weiteren Volumens platziert zu werden, um ein Bezugssignal bereitzustellen.

3. System nach Anspruch 2, **dadurch gekennzeichnet, dass** die weiteren Sensormittel weitere magnetische Mittel umfassen, die als Resonanzschaltung ausgebildet sind, wobei die weiteren magnetischen Mittel ausgebildet sind, um in dem genannten weiteren Volumen ein oszillierendes Magnetfeld zu induzieren.

4. System nach einem der vorhergehenden Ansprüche 1 bis 3, **dadurch gekennzeichnet, dass** das Zielvolumen ein Herz des Benutzers umfasst.

5. System nach einem der vorhergehenden Ansprüche 1 bis 4, **dadurch gekennzeichnet, dass** das System weiterhin Alarmmittel umfasst, die von den Detektionsmitteln betätigt werden können, wobei die Alarmmittel ausgebildet sind, bei Detektion der genannten Informationen durch die Detektionsmittel ein Alarmsignal auszulösen.

6. System nach Anspruch 5, wobei das System Übertragungsmittel umfasst, die zum Übertragen des Alarmsignals zu einer auf das genannte Alarmsignal ansprechenden Fernstation ausgebildet sind.

7. Sensormittel zur Verwendung in einem System nach einem der vorhergehenden Ansprüche, welche Sensormittel als Resonanzschaltung ausgebildete magnetische Mittel umfassen, wobei die magnetischen Mittel ausgelegt sind, in einem zu untersuchenden Volumen ein oszillierendes Magnetfeld zu induzieren, wobei die Sensormittel weiterhin ausgelegt sind, mit einem Leistungszufuhrmittel verbindbar zu sein,
**dadurch gekennzeichnet, dass** die Sensormittel in einen isolierenden Gewebeträger integriert sind, der ein Teil eines Bettlakens ist.

8. Sensormittel nach Anspruch 7, in dem der genannte Gewebeträger Gewebefäden umfasst, **dadurch gekennzeichnet, dass** die magnetischen Mittel eine Schleife aus einem leitfähigen Material umfassen, welches leitfähige Material mit den Gewebefäden verwoben ist.

## Revendications

1. Système de surveillance de l'état d'un utilisateur, ledit système comprenant :
- des moyens capteurs aménagés pour être placés au voisinage d'un volume cible de l'utilisateur pour détecter des informations représentatives de l'état de l'utilisateur, lesdits moyens capteurs comprenant des moyens magnétiques aménagés sous la forme d'un circuit résonant, lesdits moyens magnétiques étant aménagés de manière à induire un champ magnétique oscillant dans ledit volume cible,
- des moyens d'alimentation électrique qui peuvent être connectés auxdits moyens magnétiques, lesdits moyens d'alimentation électrique étant aménagés de manière à fournir un signal caractéristique d'une perte de courant dudit circuit résonant lors d'une application dudit champ magnétique oscillant audit volume cible,
- des moyens de détection actionnables par desdits moyens capteurs, lesdits moyens de détection étant aménagés de manière à traiter ledit signal pour en déduire lesdites informations,
**caractérisé en ce que** les moyens magnétiques sont intégrés à un support de tissu isolant qui fait partie d'un couvre-lit.

2. Système selon la revendication 1, **caractérisé en ce que** le système comprend d'autres moyens capteurs aménagés pour être placés au voisinage d'un autre volume afin de fournir un signal de référence.

3. Système selon la revendication 2, **caractérisé en ce que** les autres moyens capteurs comprennent d'autres moyens magnétiques aménagés sous la forme d'un circuit résonant, lesdits moyens magnétiques étant aménagés de manière à induire un champ magnétique oscillant dans ledit autre volume.

4. Système selon l'une quelconque des revendications précédentes 1 à 3, **caractérisé en ce que** le volume cible comprend le coeur d'un utilisateur.

5. Système selon l'une quelconque des revendications précédentes 1 à 4, **caractérisé en ce que** le système comprend en outre des moyens d'alarme actionnables par les moyens de détection, lesdits moyens d'alarme étant aménagés de manière à déclencher un signal d'alarme en cas de détection desdites informations par les moyens de détection.

6. Système selon la revendication 5, ledit système comprenant des moyens de transmission aménagés de manière à transmettre le signal d'alarme à un poste distant réagissant audit signal d'alarme.

7. Moyens capteurs pour usage dans un système selon l'une quelconque des revendications précédentes, lesdits moyens capteurs comprenant des moyens magnétiques aménagés sous la forme d'un circuit résonant, lesdits moyens magnétiques étant conçus de manière à induire un champ magnétique oscillant dans un volume en cours d'étude, lesdits moyens capteurs étant en outre conçus de manière à pouvoir être connectés à des moyens d'alimentation électrique, **caractérisés en ce que** lesdits moyens capteurs sont intégrés à un support de tissu isolant qui fait partie d'un drap de lit.

8. Moyens capteurs selon la revendication 7, dans lesquels ledit support de tissu comprend des fils de tissu, **caractérisés en ce que** les moyens magnétiques comprennent une boucle d'un matériau conducteur, ledit matériau conducteur étant entrelacé avec lesdits fils de tissu.
